# EUROPEAN PATENT SPECIFICATION

(11) **EP 3 813 070 B1**
(45) Date of publication and mention of the grant of the patent: **06.08.2025**
(21) Application number: 20197353.4
(22) Date of filing: 22.09.2020
(51) Int. Cl.: G16B 20/20, G16B 20/40

(54) **METHOD AND SYSTEM FOR MATCHING PHENOTYPE DESCRIPTIONS AND PATHOGENIC VARIANTS**
VERFAHREN UND SYSTEM ZUM ABGLEICH VON PHÄNOTYPBESCHREIBUNGEN UND PATHOGENEN VARIANTEN
PROCÉDÉ ET SYSTÈME DE MISE EN CORRESPONDANCE DE DESCRIPTIONS DE PHÉNOTYPES ET DE VARIANTS PATHOGÈNES

(30) Priority: 25.10.2019 IN 201921043575
(43) Date of publication of application: 28.04.2021
(73) Proprietor: Tata Consultancy Services Limited, Maharashtra (IN)
(72) Inventor: JOSEPH, Thomas, 560066 Bengaluru, Karnataka (IN); RAO, Aditya Ramkrishna, 560066 Bengaluru, Karnataka (IN); Govindakrishnan, Saipradeep Vangala, 560066 Bengaluru, Karnataka (IN); SIVADASAN, Naveen, 500081 Hyderabad - Telengana (IN); Sunderam, Uma, 600113 Taramani, Chennai (IN); KOTTE, Sujatha, 500081 Hyderabad, Telengana (IN); SRINIVASAN, Rajgopal, 500081 Hyderabad, Telengana (IN)
(74) Representative: Goddar, Heinz J.

(56) References cited:
- EP-A1- 3 550 568
- WO-A1-2015/109021
- ADITYA RAO ET AL: "Phenotype-driven gene prioritization for rare diseases using graph convolution on heterogeneous networks", BMC MEDICAL GENOMICS, vol. 11, no. 1, 6 July 2018 (2018-07-06), XP055614958, DOI: 10.1186/s12920-018-0372-8
- WILLIAM WEBBER ET AL: "A similarity measure for indefinite rankings", ACM TRANSACTIONS ON INFORMATION SYSTEMS, ASSOCIATION FOR COMPUTING MACHINERY, 2 PENN PLAZA, SUITE 701 NEW YORK NY 10121-0701 USA, vol. 28, no. 4, 23 November 2010 (2010-11-23), pages 1 - 38, XP058348009, ISSN: 1046-8188, DOI: 10.1145/1852102.1852106
- LANKTREE MATTHEW B ET AL: "Meta-analysis of Dense Genecentric Association Studies Reveals Common and Uncommon Variants Associated with Height", THE AMERICAN JOURNAL OF HUMAN GENETICS, vol. 88, 7 January 2011 (2011-01-07), pages 6 - 18, XP055778473
- ROBINSON P. N. ET AL: "Improved exome prioritization of disease genes through cross-species phenotype comparison", GENOME RESEARCH, vol. 24, no. 2, 25 October 2013 (2013-10-25), US, pages 340 - 348, XP055778864, ISSN: 1088-9051, DOI: 10.1101/gr.160325.113

## Description

### CROSS-REFERENCE TO RELATED APPLICATIONS AND PRIORITY

This patent application claims priority to India Patent Application 201921043575, filed on October 25, 2019.

### TECHNICAL FIELD

The disclosure herein generally relates to the field of phenotypegenotype matching and, more particular, to a method and system for matching phenotype descriptions and pathogenic variants.

### BACKGROUND

Diagnosis of rare human diseases using DNA sequencing is a fast growing area of research. Consequently, there has been a significant rise in the identification on novel variants with regards to both known as well as novel disease genes. This provides an understanding of the role of these genes in rare human disease. However, this is crucially dependent on rapid, comprehensive and accurate assessment of a subject's genomic variants and the relation of these variants with the observed clinical phenotypes

Conventional methods includes Whole Genome Sequencing (WGS), Whole Exome Sequencing (WES), gene panel sequencing and sequencing of specific gene(s). Other methods includes direct look-up of subject phenotypes in resources such as the Online Mendelian Inheritance in Man (OMIM) disease catalog which helps to filter variant lists by limiting the search to genes already known to contain variants associated with a set of phenotypes. However, this carries a risk of incorrect phenotype interpretation. To overcome this, other sources of information have been used such as phenotype information of other organisms to extrapolate human gene phenotype associations. However, obtaining a correct genotype and phenotype matching is challenging.

WO 2015/109021 A1 discloses methods and systems for prioritizing phenotype-causing genomic variants. The methods include using variant prioritization analyses and in combination with biomedical ontologies using a sophisticated reranking methodology to re-rank these variants based on phenotype information. The methods can be useful in any genomics study and diagnostics; for example, rare and common disease gene discovery, tumor growth mutation detection, drug responder studies, metabolic studies, personalized medicine, agricultural analysis, and centennial analysis.

### SUMMARY

Embodiments of the present disclosure present technological improvements as solutions to one or more of the above-mentioned technical problems recognized by the inventors in conventional systems. In one embodiment, a method for matching phenotype descriptions and pathogenic variants is provided. The invention is set out in the appended set of claims 1-4.

In another aspect, a system for matching phenotype descriptions and pathogenic variants is provided. The invention is set out in the appended set of claims 5-8.

In yet another aspect, a computer program product including a non-transitory computer-readable medium having embodied therein a computer program for method and system for matching phenotype descriptions and pathogenic variants is provided. The invention is set out in the appended claim 9.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 is a functional block diagram of a system for matching phenotype descriptions and pathogenic variants, according to some embodiments of the present disclosure.
FIG. 2A, 2B and 2C are schematic block diagrams illustrating a method for matching phenotype descriptions and pathogenic variants, according to some embodiments of the present disclosure.
FIG. 3 is an exemplary flow diagrams for a processor implemented method for matching phenotype descriptions and pathogenic variants, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts.

Embodiments herein provide a method and system for matching phenotype descriptions and pathogenic variants. The system for matching phenotype descriptions and pathogenic variants provides a one to one mapping of the phenotype and genotypes of a plurality of subjects under test. A plurality of phenotypes and a plurality of genome sequences are segmented based on metadata. The segmented phenotype data is further processed by a phenotype driven gene prioritization. The segmented genotype sequences are processed simultaneously by a variant prioritization method. The output of the phenotype driven gene prioritization and the variant prioritization are utilized to compute a similarity score. The similarity score is further utilized to obtain a one to one matching of the plurality of phenotypes and the plurality of genotype sequences of the plurality of subjects under test.

The similarity score is calculated by utilizing a rank biased overlapping and the one to one mapping is performed by utilizing a Hungarian matching algorithm. The said matching techniques are used for matching data in other domains and hardly any attempts to use in the domain of phenotype to genotype matching. The present disclosure applies the said matching techniques in phenotype to genotype matching to improve the accuracy of matching. Here, the similarity score provides a list of possible matches and the corresponding ranking score. Further application of the Hungarian matching algorithm on the list of possible matches provides the accurate one to one matching.

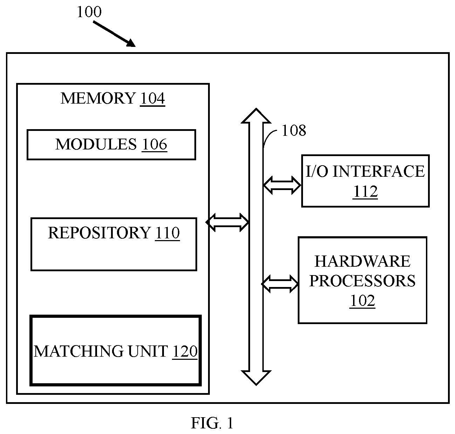

Referring now to the drawings, and more particularly to FIG. 1 through 3, where similar reference characters denote corresponding features consistently throughout the figures, there are shown preferred embodiments and these embodiments are described in the context of the following exemplary system and/or method.

FIG. 1 is a functional block diagram of a system for matching phenotype descriptions and pathogenic variants, according to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the interface 112 may enable the system 100 to communicate with other devices, such as web servers and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting a number of computing systems with one another or to another server computer. The I/O interface 112 may include one or more ports for connecting a number of devices to one another or to another server.

The one or more hardware processors 102 may be implemented as one or more microprocessors, microcomputers, microcontrollers, digital signal processors, central processing units, state machines, logic circuitries, and/or any devices that manipulate signals based on operational instructions. Among other capabilities, the hardware processor 102 is configured to fetch and execute computer-readable instructions stored in the memory 104.

The memory 104 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 104 includes a plurality of modules 106, a matching unit 120. The memory 104 also includes a repository 110 for storing data processed, received, and generated by one or more of the modules 106 and the matching unit 120. The modules 106 may include routines, programs, objects, components, data structures, and so on, which perform particular tasks or implement particular abstract data types.

The memory 104 also includes module(s) 106 and a data repository 110. The module(s) 106 include programs or coded instructions that supplement applications or functions performed by the system 100 for matching phenotype descriptions and pathogenic variants. The modules 106, amongst other things, can include routines, programs, objects, components, and data structures, which perform particular tasks or implement particular abstract data types. The modules 106 may also be used as, signal processor(s), state machine(s), logic circuitries, and/or any other device or component that manipulates signals based on operational instructions. Further, the modules 106 can be used by hardware, by computer-readable instructions executed by a processing unit, or by a combination thereof. The modules 106 can include various sub-modules (not shown). The modules 106 may include computer-readable instructions that supplement applications or functions performed by the system 100 for matching phenotype descriptions and pathogenic variants.

The data repository 110 may include a database of Electronic Medical Record (EMR)/ Electronic Health Record (EHR) and other data. Further, the other data amongst other things, may serve as a repository for storing data that is processed, received, or generated as a result of the execution of one or more modules in the module(s) 106 and the modules associated with the matching unit 120.

Although the repository 110 is shown internal to the system 100, it will be noted that, in alternate embodiments, the repository 110 can also be implemented external to the computing device 100, where the repository 110 may be stored within a database (not shown in FIG. 1) communicatively coupled to the system 100. The data contained within such external database may be periodically updated. For example, new data may be added into the database (not shown in FIG. 1) and/or existing data may be modified and/or non-useful data may be deleted from the database (not shown in FIG. 1). In one example, the data may be stored in an external system, such as a Lightweight Directory Access Protocol (LDAP) directory and a Relational Database Management System (RDBMS).

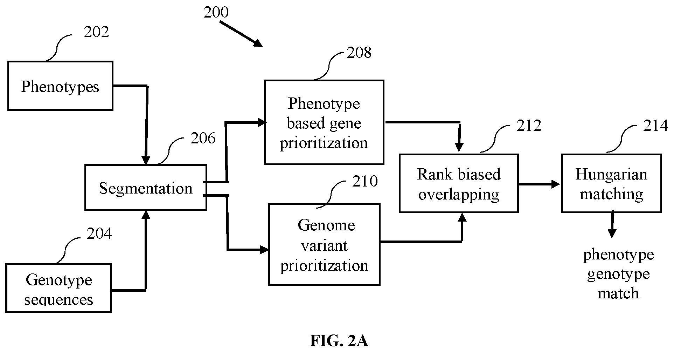

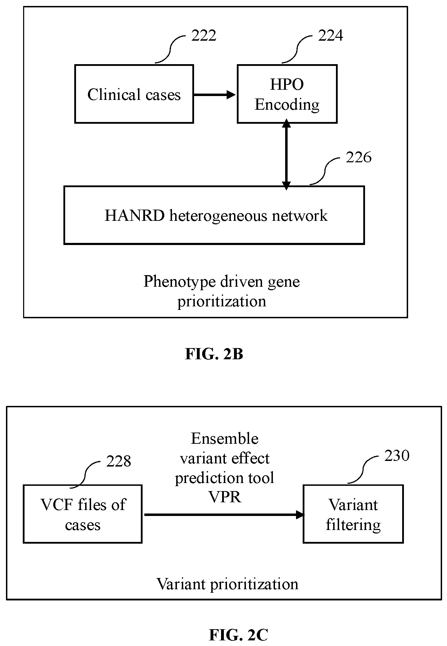

FIG. 2A is a schematic block diagram illustrating the method 200 for matching phenotype descriptions and pathogenic variants, according to some embodiments of the present disclosure. Now referring to FIG. 2A, the method 200 receives a plurality of phenotypes 202 and a plurality of genotype sequences 204. Further, segmentation is performed on the plurality of phenotypes and the plurality of genotype sequences based on a plurality of metadata by a segmentation module 206. The segmented phenotypes are further converted into a first list of ranked potential causal genes by a phenotype based gene prioritization module 208 (described in FIG. 2B). The segmented genome sequences are further converted into a second list of ranked potential causal genes by a genome variant prioritization module 210 (described in FIG. 2C). A similarity measure between each of the first list of ranked potential causal genes and each of the second list of ranked potential causal genes is calculated by a rank biased overlapping module 212. The Hungarian matching module 214 performs a one to one genotype-phenotype matching corresponding to each of the plurality of subjects under test based on the corresponding similarity measure.

The matching unit 120, executed by the one or more processors of the system 100, receives the plurality of phenotypes and the plurality of genotype sequences pertaining to a plurality of subjects under test. For example, the plurality of phenotypes includes fever, cardiomyopathy, muscular dystrophy and rhabdomyolysis. The plurality of genotype sequences includes raw DNA sequences and Variant Call Format (VCF) files. The VCF specifies the format of a text file used in bioinformatics for storing gene sequence variations.

Further, the matching unit 120, executed by the one or more processors of the system 100, segments the plurality of phenotypes and the plurality of genotype sequences based on a plurality of metadata, wherein the plurality of metadata include a gender, age group, socioeconomic status, region, family history and an ethnicity associated with the subject under test.

In an embodiment, the method of segmenting the plurality of phenotypes based on the plurality of metadata includes (i) segmenting the plurality of phenotypes to obtain a set of male phenotypes and a set of female phenotypes based on the gender (ii) segmenting the set of male phenotypes into a plurality of male ethnic phenotypes based on the ethnicity and (iii) simultaneously segmenting the set of female phenotypes into a plurality of female ethnic phenotypes based on the ethnicity.

In an embodiment, the method of segmenting the plurality of genotype sequences based on the plurality of metadata includes (i) segmenting the plurality of genotype sequences to obtain a set of male genotypes and a set of female genotypes based on the gender (ii) segmenting the set of male genotypes into a plurality of male ethnic genotypes based on the ethnicity and (iii) simultaneously segmenting the set of female genotypes into a plurality of female ethnic genotypes based on the ethnicity.

Further, the matching unit 120, executed by one or more processors of the system 100, computes a first list of ranked potential causal genes for the plurality of segmented phenotypes based on a phenotype based gene prioritization, wherein the first list of ranked potential causal genes is associated with corresponding phenotypes from the set of segmented phenotypes. A variant of the potential causal gene is likely to cause a set of phenotypes corresponding to a reference subject.

FIG. 2B is a schematic block diagram illustrating the method for phenotype driven gene prioritization, according to some embodiments of the present disclosure. Now referring to FIG. 2B, each phenotype from the plurality of phenotypes 222 is associated with an initial Human Phenotype Ontology (HPO) code 224. Initially, the plurality of segmented phenotypes are provided as input to a phenotype tagger. The phenotype tagger utilizes a phenotype dictionary. The Phenotype dictionary used by the phenotype tagger was derived primarily from semi-automated curation of data from HPO augmented with MeSH terms. The phenotype tagger provides additional HPO codes as output. The additional HPO codes were augmented with the initial HPO codes to obtain the HPO codes. Further, the HPO codes are utilized to query a Heterogeneous Association Network for Rare Diseases (HANRD) heterogeneous network 226 to obtain a ranked list of genes associated with the case's phenotypes. The HANRD is a heterogeneous network including entities such as genes, phenotypes, diseases and pathways as nodes while associations between these entities are represented as weighted edges, with the edges representing curated as well as inferred associations. The score of the association between the entity pairs is represented as the edge weight. The HANRD operates by combining a pairwise ontological and curated associations into a single heterogeneous association network.

Further, the HANRD network is augmented with additional inferred associations computed using the information propagation algorithm GCAS (Graph Convolution-based Association Scoring). GCAS performs information propagation on the ontological and curated association network to infer novel associations between the entities of this network. For each case's 222 input set of HPO codes, the output is a prioritized gene and disease list. Even though the primary gene lists were from HANRD, Phenomizer gene list is also utilized as an additional source to obtain potential genes linked to the input phenotype. Similar to HANRD, Phenomizer takes the HPO codes of each phenotype case 222 as input and ranks diseases by a score that reflects how well the phenotype profiles of the case and the disease match. The output of Phenomizer is a prioritized disease list with each disease having any associated gene(s) mentioned in a separate column. In an embodiment, a Genetic and Rare Diseases database (GARD) gene list is also utilized to analyze phenotypes for a disease. The GARD has a frequency based classification of the plurality of phenotypes into a plurality of classes like 80% to 90% of the subjects having the phenotypes, 30% to 79% of the subjects having the phenotypes and a percentage of the subjects whose symptoms were not listed in the HPO.

Further, the matching unit 120, executed by one or more processors of the system 100, simultaneously computes the second list of ranked potential causal genes for the plurality of segmented genotypes based on a genome variant prioritization, wherein the genome variant prioritization analyzes, annotates and prioritize genomic variants.

FIG. 2C is a schematic block diagram illustrating the method for variant prioritization, according to some embodiments of the present disclosure. The variant prioritization ranks a variant observed in an individual's genome on the basis of factors including a predicted consequence of each variant and an observed frequency in populations. The variant prioritization typically identifies a few hundred variants in a subject under test.

In an embodiment, the variant prioritization module (as explained in FIG. 2C) includes a conservation annotation and a functional annotation. A plurality of genotype VCF files 228 is provided as input to an ensemble variant effect prioritization pipeline, referred to as VPR. Here, variants are prioritized using an internal scoring scheme, ranging from 0 to 1 and represents a weighted combinations of conservation and functional scores. The conservation scores are derived using GERP (Genomic Evolutionary Rate Profiling), SiPhy, PhastCons (primate, vertebrate and mammalian data) and PhyloP (primate and vertebrate data). The frequency of the variant in the 1000 Genomes and GnomAD databases is also considered. The conservation scores were used to internally classify the variant as one of damaging, possibly damaging or tolerated, based on thresholds derived from the ClinVar database. The functional scores were derived from different variant effect predictor tools. Raw scores from tools were internally classified as being damaging, possibly damaging or tolerated based on thresholds derived from ClinVar database and a collective voting scheme has been utilized to arrive at the functional score for a given variant based on the region and variant type. For example, the score for a missense Single Nucleotide Variant (SNV) was derived from the combined deleteriousness vote of tools including Combined Annotation Dependent Depletion (CADD), SIFTok, PolyPhen-2 37 and REVEL. For PolyPhen-2, we used both PolyPhen-2_{HDIV} to identify damaging variants by assuming that the differences between human proteins and their closely related mammalian homologs will be non-damaging, as well as PolyPhen-2_{HVAR} to identify disease-causing variants by assuming common human nsSNPs (non-synchronous Single Nucleotide Polymorphisms) to be non-damaging.

The variant score thus derived from conservation and functional annotations ranged between 0 and 1, with 1 being the most likely damaging. Each gene was scored as the maximum additive score from two independent variants (or homozygous variant) that occurred in the same gene for a particular sample with the assumption of a recessive model.

The variant filtering module 230 of FIG. 2C performs filtering based on a threshold as explained below: In an embodiment, a plurality of high quality variants were selected by including the variants having PASS and variant score threshold of >=0.69. In addition, the variants occurred in polymorphic genes were excluded. Further, the genes are ranked based on the variants by combining the scores of the top two scoring variants in the same gene for the recessive (AR) inheritance model and selected the gene with the top scoring variant for the dominant (AD) inheritance model. Finally the second list of potential causal genes and corresponding Diagnostic Variants (DVs) for each genotype case file is generated.

Further, the matching unit 120, executed by one or more processors of the system 100, computes a similarity measure between each of the first list of ranked potential causal genes and each of the second list of ranked potential causal genes based on a rank biased overlapping. For example, the similarity measure can be calculated by utilizing any one of the known techniques including Kendall's τ, Spearman's ρ, Spearman's footrule, Goodman and Kruskal's γ and AnchorMAP.

The computation of similarity score includes a plurality of characteristics like top-weightedness, non-conjointness, indefiniteness and rank ties. The top weightedness characteristics says that the genes that are ranked on top of the lists should have higher score than the ones ranked low in the lists. The non-conjointness characteristics says that the genes present in the variant prioritized list may not be present in the phenotype prioritized list, or vice versa. The indefiniteness characteristics says that, the size of ranked gene list from phenotype analysis might differ from the genotype analysis and thus the overlapping gene(s) can be found at varying depths in the lists. The rank ties characteristics says that the ranked gene lists can have the same ranks (ties) for those genes with similar prioritization scores.

The Rank Biased Overlap (RBO) provides the similarity measure, satisfying the plurality of above characteristics. In RBO, a set-based similarity measure, provides a bounded similarity measure between two lists by calculating the average overlap score at each depth on the entire ranked gene list(s) using a convergent series of weights. RBO is top-weighted and handles non-conjoint, indefinite lists with ties.

Further, the matching unit 120, executed by one or more processors of the system 100, matches each of the first list of ranked potential causal genes and each of the second list of ranked potential causal genes to obtain a plurality of one to one genotype-phenotype match corresponding to each of the plurality of subjects under test based on the corresponding similarity measure. The one-to-one (perfect) matching between cases is obtained by utilizing the "Hungarian matching" algorithm. The Hungarian matching algorithm computes perfect match by finding an optimal assignment which maximizes the (cost) similarity measure between the genotype and phenotype cases.

In an embodiment an example matching is described here: consider two subjects named a subject 1 and a subject 2. The plurality of phenotypes associated with the subject 1 is (P1, P2, P3, P4) and subject 2 is (P2, P4, P5, P6) respectively. The corresponding two genotype VCF files are VCF file A and VCF file B. The associations of the VCF files to the subjects are unknown and needed to find the association between the two genotype files and the two subjects. There are two possible associations. One possibility is that the VCF file A is associated with Subject 1 and VCF file B is associated with Subject 2. The second possibility is that VCF file A is associated with Subject 2 and the VCF file B is associated with Subject 1. In order to find the correct association among the two possibilities, two separate lists of prioritized genes (the first list of ranked potential causal genes) are obtained separately for Subjects 1 and 2 based on their phenotypes. Also, two separate lists of prioritized gene lists (the second list of ranked potential causal genes) are obtained by analyzing the subject VCF file A and VCF file B using variant prioritization. Further, matching between the first lists of potential causal genes and the second lists of potential causal genes is explained here: There are two gene prioritized lists (the first list of ranked potential causal genes), one for Subject 1 and the other for Subject 2 in one side. Also, there are two gene prioritized lists (the second list of ranked potential causal genes), one for VCF A and the other for VCF B on the other side. Between every pair of the gene lists, where one list is from one side and the other list is from the other side, an overlap (similarity) score between the two lists is calculated. Further a matching (one-to-one marriage) between the two sides are performed, where each entry on side one is matched with a distinct entry on the other side, in such a way that, the cumulative overlap (similarity) scores of the matched partners is maximum. The Hungarian matching algorithm is utilized to compute one to one matching. The resulting matching directly gives the desired association between the two subjects and the two VCF files.

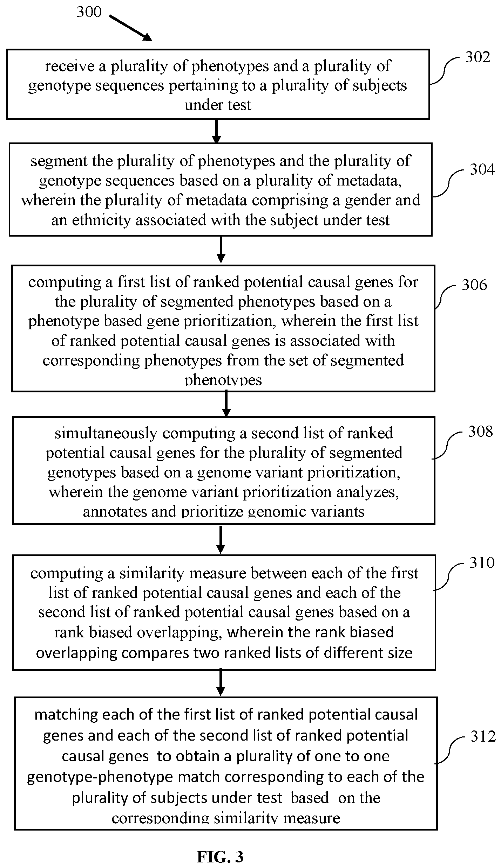

FIG. 3 is an exemplary flow diagrams for a processor implemented method for matching phenotype descriptions and pathogenic variants, according to some embodiments of the present disclosure. The method 300 may be described in the general context of computer executable instructions. Generally, computer executable instructions can include routines, programs, objects, components, data structures, procedures, modules, functions, etc., that perform particular functions or implement particular abstract data types. The method 300 may also be practiced in a distributed computing environment where functions are performed by remote processing devices that are linked through a communication network. The order in which the method 300 is described is not intended to be construed as a limitation, and any number of the described method blocks can be combined in any order to implement the method 300, or an alternative method. Furthermore, the method 300 can be implemented in any suitable hardware, software, firmware, or combination thereof.

At 302, the method 300, receives, by a one or more hardware processors, the plurality of phenotypes and the plurality of genotype sequences pertaining to a plurality of subjects under test.

At 304, the method 300, segments, by a one or more hardware processors, the plurality of phenotypes and the plurality of genotype sequences based on the plurality of metadata. The plurality of metadata includes the gender and the ethnicity associated with the subject under test. The method of segmenting the plurality of phenotypes based on the plurality of metadata includes (i) segmenting the plurality of phenotypes to obtain a set of male phenotypes and a set of female phenotypes based on the gender (ii) segmenting the set of male phenotypes into a plurality of male ethnic phenotypes based on the ethnicity and (iii) simultaneously segmenting the set of female phenotypes into a plurality of female ethnic phenotypes based on the ethnicity. The method of segmenting the plurality of genotype sequences based on the plurality of metadata includes (i) segmenting the plurality of genotype sequences to obtain a set of male genotypes and a set of female genotypes based on the gender (ii) segmenting the set of male genotypes into a plurality of male ethnic genotypes based on the ethnicity and (iii) simultaneously segmenting the set of female genotypes into a plurality of female ethnic genotypes based on the ethnicity.

At 306, the method 300, computes, by a one or more hardware processors, the first list of ranked potential causal genes for the plurality of segmented phenotypes based on the phenotype based gene prioritization. The first list of ranked potential causal genes is associated with corresponding phenotypes from the set of segmented phenotypes.

At 308, the method 300, simultaneously computes, by a one or more hardware processors, the second list of ranked potential causal genes for the plurality of segmented genotypes based on the genome variant prioritization, wherein the genome variant prioritization analyzes, annotates and prioritize genomic variants

At 310, the method 300, computes, by a one or more hardware processors, the similarity measure between each of the first list of ranked potential causal genes and each of the second list of ranked potential causal genes based on the rank biased overlapping, wherein the rank biased overlapping compares two ranked lists of different size.

At 312, the method 300, matches, by a one or more hardware processors, each of the first list of ranked potential causal genes and each of the second list of ranked potential causal genes to obtain the plurality of one to one genotype-phenotype match corresponding to each of the plurality of subjects under test based on the corresponding similarity measure

In an embodiment, the system 100 is experimented as follows: The system 100 is experimented on "SickKids Clinical Genomes Challenge" database from the SickKids Genome Clinic, Toronto, Canada. The experimentation involved 24 children (hereinafter referred to as 'the subjects') who were referred for clinical Whole Genome Sequencing (WGS) with suspected rare genetic diseases. The genome sequences of the 24 subjects and the associated plurality of phenotypes of the 24 subjects were provides separately. The main aim of the experimentation was to predict the class of disease associated with each genome, and which genome corresponds to which specific clinical description of clinical phenotypes. The predictors could specify the probable causal variant(s) underlying the match predictions, referred to as disease variants (DVs). The predictors also could identify predictive secondary variants (PSVs) that could confer high risk of other diseases whose phenotypes are not included in the clinical descriptions.

The written description describes the subject matter herein to enable any person skilled in the art to make and use the embodiments.

The embodiments of present disclosure herein addresses unresolved problem of generating the plurality of test cases for complex domains. Here, the system 100 matches gene lists from variant prioritization along with those from phenotypedriven analysis using an automated matching algorithm. The automated matching algorithm provides significantly better matching.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an applicationspecific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e. non-transitory. Examples include random access memory (RAM), readonly memory (ROM), volatile memory, nonvolatile memory, hard drives, CD ROMs, DVDs, flash drives, disks, and any other known physical storage media.

## Claims

1. A processor-implemented method for matching clinical phenotype descriptions and pathogenic variants, the method comprising:
receiving, by one or more hardware processors, a plurality of phenotypes and a plurality of genotype sequences pertaining to a plurality of subjects under test (302);
segmenting, by the one or more hardware processors, the plurality of phenotypes and the plurality of genotype sequences based on a plurality of metadata, wherein the plurality of metadata comprising a gender and an ethnicity associated with the subject under test (304);
computing, by the one or more hardware processors, a first list of ranked potential causal genes for the plurality of segmented phenotypes based on a phenotype based gene prioritization, wherein the first list of ranked potential causal genes is associated with corresponding phenotypes from the set of segmented phenotypes(306), wherein computing the first list of ranked potential causal genes based on the phenotype based gene prioritization comprises the steps of:
providing the plurality of segmented phenotypes as an input to a phenotype tagger, wherein each phenotype from the plurality of phenotypes is associated with an initial Human Phenotype Ontology, HPO, code;
providing additional HPO codes as output by the phenotype tagger by using a phenotype dictionary;
augmenting the additional HPO codes with the initial HPO codes to obtain HPO codes; and
querying a Heterogeneous Association Network for Rare Diseases, HANRD, heterogeneous network by utilizing the HPO codes to obtain the first list of ranked potential causal genes associated with the corresponding phenotypes from the set of segmented phenotypes;
simultaneously computing, by the one or more hardware processors, a second list of ranked potential causal genes for the plurality of segmented genotypes based on a genome variant prioritization, wherein the genome variant prioritization analyzes, annotates and prioritize genomic variants (308), wherein computing the second list of ranked potential causal genes based on the genome variant prioritization comprises the steps of:
providing a plurality of genotype Variant Call Format, VCF, files as an input to an ensemble variant effect prioritization pipeline;
deriving a variant score by prioritizing variants using an internal scoring scheme, ranging from 0 to 1 and represents a weighted combinations of conservation and functional scores, wherein the conservation scores used to internally classify a variant as one of damaging, possibly damaging or tolerated, based on thresholds derived from a ClinVar database; and
performing the variant filtering based on a variant score threshold, wherein the variant filtering comprises:
selecting a plurality of high quality variants by including the variants having PASS and the variant score threshold;
eliminating the variants occurred in polymorphic genes;
ranking the genes based on the variants by combining scores of the top two scoring variants in a same gene for a recessive inheritance model and selecting the gene with a top scoring variant for the dominant inheritance model; and
generating the second list of potential causal genes for the plurality of segmented genotypes based on the ranking;
computing, by the one or more hardware processors, a similarity measure between each of the first list of ranked potential causal genes and each of the second list of ranked potential causal genes based on a rank biased overlapping, wherein the rank biased overlapping compares two ranked lists of different size (310); and
matching, by the one or more hardware processors, each of the first list of ranked potential causal genes and each of the second list of ranked potential causal genes by using a Hungarian matching algorithm to obtain a plurality of one to one genotype-phenotype match corresponding to each of the plurality of subjects under test based on the corresponding similarity measure, wherein the Hungarian matching algorithm improves accuracy of matching by computing a match by finding an optimal assignment which maximizes the similarity measure between the genotype and phenotype cases (312).

2. The method of claim 1, wherein segmenting the plurality of phenotypes based on the plurality of metadata comprising:
segmenting the plurality of phenotypes to obtain a set of male phenotypes and a set of female phenotypes based on the gender;
segmenting the set of male phenotypes into a plurality of male ethnic phenotypes based on the ethnicity; and
simultaneously segmenting the set of female phenotypes into a plurality of female ethnic phenotypes based on the ethnicity.

3. The method of claim 1, wherein segmenting the plurality of genotype sequences based on the plurality of metadata comprising:
segmenting the plurality of genotype sequences to obtain a set of male genotypes and a set of female genotypes based on the gender;
segmenting the set of male genotypes into a plurality of male ethnic genotypes based on the ethnicity; and
simultaneously segmenting the set of female genotypes into a plurality of female ethnic genotypes based on the ethnicity.

4. The method of claim 1, wherein a variant of the potential causal gene is likely to cause a set of phenotypes corresponding to a reference subject.

5. A system (100) for matching clinical phenotype descriptions and pathogenic variants, comprising:
at least one memory (104) storing programmed instructions;
one or more Input /Output (I/O) interfaces (112); and
one or more hardware processors (102) operatively coupled to the at least one memory (104), wherein the one or more hardware processors (102) are configured by the programmed instructions to:
receive a plurality of phenotypes and a plurality of genotype sequences pertaining to a plurality of subjects under test;
segment the plurality of phenotypes and the plurality of genotype sequences based on a plurality of metadata, wherein the plurality of metadata comprising a gender and an ethnicity associated with the subject under test;
compute a first list of ranked potential causal genes for the plurality of segmented phenotypes based on a phenotype based gene prioritization, wherein the first list of ranked potential causal genes is associated with corresponding phenotypes from the set of segmented phenotypes, wherein computing the first list of ranked potential causal genes based on the phenotype based gene prioritization comprises the steps of:
providing the plurality of segmented phenotypes as an input to a phenotype tagger, wherein each phenotype from the plurality of phenotypes is associated with an initial Human Phenotype Ontology, HPO, code;
providing additional HPO codes as output by the phenotype tagger by using a phenotype dictionary;
augmenting the additional HPO codes with the initial HPO codes to obtain HPO codes; and
querying a Heterogeneous Association Network for Rare Diseases, HANRD, heterogeneous network by utilizing the HPO codes to obtain the first list of ranked potential causal genes associated with the corresponding phenotypes from the set of segmented phenotypes;
simultaneously compute a second list of ranked potential causal genes for the plurality of segmented genotypes based on a genome variant prioritization, wherein the genome variant prioritization analyzes, annotates and prioritize genomic variants, wherein computing the second list of ranked potential causal genes based on the genome variant prioritization comprises the steps of:
providing a plurality of genotype Variant Call Format, VCF, files as an input to an ensemble variant effect prioritization pipeline;
deriving a variant score by prioritizing variants using an internal scoring scheme, ranging from 0 to 1 and represents a weighted combinations of conservation and functional scores, wherein the conservation scores used to internally classify a variant as one of damaging, possibly damaging or tolerated, based on thresholds derived from a ClinVar database; and
performing the variant filtering based on a variant score threshold, wherein the variant filtering comprises:
selecting a plurality of high quality variants by including the variants having PASS and the variant score threshold;
eliminating the variants occurred in polymorphic genes;
ranking the genes based on the variants by combining scores of the top two scoring variants in a same gene for a recessive inheritance model and selecting the gene with a top scoring variant for the dominant inheritance model; and
generating the second list of potential causal genes for the plurality of segmented genotypes based on the ranking;
compute a similarity measure between each of the first list of ranked potential causal genes and each of the second list of ranked potential causal genes based on a rank biased overlapping, wherein the rank biased overlapping compares two ranked lists of different size; and
match each of the first list of ranked potential causal genes and each of the second list of ranked potential causal genes by using a Hungarian matching algorithm to obtain a plurality of one to one genotype-phenotype match corresponding to each of the plurality of subjects under test based on the corresponding similarity measure, wherein the Hungarian matching algorithm improves accuracy of matching by computing a match by finding an optimal assignment which maximizes the similarity measure between the genotype and phenotype cases .

6. The system as claimed in claim 5, wherein segmenting the plurality of phenotypes based on the plurality of metadata comprising:
segmenting the plurality of phenotypes to obtain a set of male phenotypes and a set of female phenotypes based on the gender;
segmenting the set of male phenotypes into a plurality of male ethnic phenotypes based on the ethnicity; and
simultaneously segmenting the set of female phenotypes into a plurality of female ethnic phenotypes based on the ethnicity.

7. The system of claim 5, wherein segmenting the plurality of genotype sequences based on the plurality of metadata comprising:
segmenting the plurality of genotype sequences to obtain a set of male genotypes and a set of female genotypes based on the gender;
segmenting the set of male genotypes into a plurality of male ethnic genotypes based on the ethnicity; and
simultaneously segmenting the set of female genotypes into a plurality of female ethnic genotypes based on the ethnicity.

8. The system of claim 5, wherein a variant of the potential causal gene is likely to cause a set of phenotypes corresponding to a reference subject.

9. One or more non-transitory machine readable information storage mediums comprising one or more instructions which when executed by one or more hardware processors causes:
receiving, by one or more hardware processors, a plurality of phenotypes and a plurality of genotype sequences pertaining to a plurality of subjects under test;
segmenting, by the one or more hardware processors, the plurality of phenotypes and the plurality of genotype sequences based on a plurality of metadata, wherein the plurality of metadata comprising a gender and an ethnicity associated with the subject under test;
computing, by the one or more hardware processors, a first list of ranked potential causal genes for the plurality of segmented phenotypes based on a phenotype based gene prioritization, wherein the first list of ranked potential causal genes is associated with corresponding phenotypes from the set of segmented phenotypes, wherein computing the first list of ranked potential causal genes based on the phenotype based gene prioritization comprises the steps of:
providing the plurality of segmented phenotypes as an input to a phenotype tagger, wherein each phenotype from the plurality of phenotypes is associated with an initial Human Phenotype Ontology, HPO, code;
providing additional HPO codes as output by the phenotype tagger by using a phenotype dictionary;
augmenting the additional HPO codes with the initial HPO codes to obtain HPO codes; and
querying a Heterogeneous Association Network for Rare Diseases, HANRD, heterogeneous network by utilizing the HPO codes to obtain the first list of ranked potential causal genes associated with the corresponding phenotypes from the set of segmented phenotypes;
simultaneously computing, by the one or more hardware processors, a second list of ranked potential causal genes for the plurality of segmented genotypes based on a genome variant prioritization, wherein the genome variant prioritization analyzes, annotates and prioritize genomic variants, wherein computing the second list of ranked potential causal genes based on the genome variant prioritization comprises the steps of:
providing a plurality of genotype Variant Call Format, VCF, files as an input to an ensemble variant effect prioritization pipeline;
deriving a variant score by prioritizing variants using an internal scoring scheme, ranging from 0 to 1 and represents a weighted combinations of conservation and functional scores, wherein the conservation scores used to internally classify a variant as one of damaging, possibly damaging or tolerated, based on thresholds derived from a ClinVar database; and
performing the variant filtering based on a variant score threshold, wherein the variant filtering comprises:
selecting a plurality of high quality variants by including the variants having PASS and the variant score threshold;
eliminating the variants occurred in polymorphic genes;
ranking the genes based on the variants by combining scores of the top two scoring variants in a same gene for a recessive inheritance model and selecting the gene with a top scoring variant for the dominant inheritance model; and
generating the second list of potential causal genes for the plurality of segmented genotypes based on the ranking;
computing, by the one or more hardware processors, a similarity measure between each of the first list of ranked potential causal genes and each of the second list of ranked potential causal genes based on a rank biased overlapping, wherein the rank biased overlapping compares two ranked lists of different size; and
matching, by the one or more hardware processors, each of the first list of ranked potential causal genes and each of the second list of ranked potential causal genes by using a Hungarian matching algorithm to obtain a plurality of one to one genotype-phenotype match corresponding to each of the plurality of subjects under test based on the corresponding similarity measure, wherein the Hungarian matching algorithm improves accuracy of matching by computing a match by finding an optimal assignment which maximizes the similarity measure between the genotype and phenotype cases .

## Patentansprüche

1. Prozessorimplementiertes Verfahren zum Abgleichen klinischer Phänotypbeschreibungen und pathogener Varianten, wobei das Verfahren Folgendes umfasst:
Empfangen, durch einen oder mehrere Hardwareprozessoren, einer Mehrzahl von Phänotypen und einer Mehrzahl von Genotypsequenzen, die sich auf eine Mehrzahl von Testpersonen (302) beziehen;
Segmentieren, durch den einen oder die mehreren Hardwareprozessoren, der Mehrzahl von Phänotypen und der Mehrzahl von Genotypsequenzen basierend auf einer Mehrzahl von Metadaten, wobei die Mehrzahl von Metadaten ein Geschlecht und eine Ethnizität umfasst, die mit der Testperson (304) assoziiert sind;
Berechnen, durch den einen oder die mehreren Hardwareprozessoren, einer ersten Liste von eingestuften potenziellen kausalen Genen für die Mehrzahl von segmentierten Phänotypen basierend auf einer phänotypbasierten Genpriorisierung, wobei die erste Liste von eingestuften potenziellen kausalen Genen mit entsprechenden Phänotypen aus dem Satz von segmentierten Phänotypen (306) assoziiert ist, wobei das Berechnen der ersten Liste von eingestuften potenziellen kausalen Genen basierend auf der phänotypbasierten Genpriorisierung die folgenden Schritte umfasst:
Bereitstellen der Mehrzahl von segmentierten Phänotypen als eine Eingabe in einen Phänotyp-Tagger, wobei jeder Phänotyp aus der Mehrzahl von Phänotypen mit einem anfänglichen menschlichen Phänotyp-Ontologie-, (Human Phenotype Ontology) HPO-, Code assoziiert ist;
Bereitstellen zusätzlicher HPO-Codes als Ausgabe durch den Phänotyp-Tagger unter Verwendung eines Phänotyp-Wörterbuchs;
Erweitern der zusätzlichen HPO-Codes mit den anfänglichen HPO-Codes, um HPO-Codes zu erhalten; und
Abfragen eines heterogenen Assoziationsnetzwerks für seltene Krankheiten (Heterogeneous Association Network for Rare Diseases), HANRD, durch Verwenden der HPO-Codes, um die erste Liste von eingestuften potenziellen kausalen Genen zu erhalten, die mit den entsprechenden Phänotypen aus dem Satz von segmentierten Phänotypen assoziiert sind;
gleichzeitiges Berechnen, durch den einen oder die mehreren Hardwareprozessoren, einer zweiten Liste von eingestuften potenziellen kausalen Genen für die Mehrzahl von segmentierten Genotypen basierend auf einer Genomvariantenpriorisierung, wobei die Genomvariantenpriorisierung genomische Varianten analysiert, annotiert und priorisiert (308), wobei das Berechnen der zweiten Liste von eingestuften potenziellen kausalen Genen basierend auf der Genomvariantenpriorisierung die folgenden Schritte umfasst:
Bereitstellen einer Mehrzahl von Genotypvariantenaufrufformat-(Variant Call Format), VCF-, Dateien als eine Eingabe in eine Ensemble-Varianteneffekt-Priorisierungspipeline;
Ableiten einer Variantenbewertung durch Priorisieren von Varianten unter Verwendung eines internen Bewertungsschemas, das von 0 bis 1 reicht und gewichtete Kombinationen von Konservierungs- und Funktionsbewertungen darstellt, wobei die Konservierungsbewertungen verwendet werden, um eine Variante intern als eine von schädlich, möglicherweise schädlich oder toleriert zu klassifizieren, basierend auf Schwellenwerten, die von einer ClinVar-Datenbank abgeleitet werden; und
Durchführen der Variantenfilterung basierend auf einer Variantenbewertungsschwelle, wobei die Variantenfilterung Folgendes umfasst:
Auswählen einer Mehrzahl von hochwertigen Varianten durch Einbeziehen der Varianten mit PASS und der Variantenbewertungsschwelle;
Eliminieren der Varianten, die in polymorphen Genen aufgetreten sind;
Einstufen der Gene basierend auf den Varianten durch Kombinieren von Bewertungen der beiden besten Bewertungsvarianten in einem gleichen Gen für ein rezessives Vererbungsmodell und Auswählen des Gens mit einer besten Bewertungsvariante für das dominante Vererbungsmodell; und
Erzeugen der zweiten Liste von potenziellen kausalen Genen für die Mehrzahl von segmentierten Genotypen basierend auf der Einstufung;
Berechnen, durch den einen oder die mehreren Hardwareprozessoren, eines Ähnlichkeitsmaßes zwischen jedem aus der ersten Liste von eingestuften potenziellen kausalen Genen und jedem aus der zweiten Liste von eingestuften potenziellen kausalen Genen basierend auf einer ranggesteuerten Überlappung, wobei die ranggesteuerte Überlappung zwei eingestufte Listen unterschiedlicher Größe vergleicht (310); und
Abgleichen, durch den einen oder die mehreren Hardwareprozessoren, jedes aus der ersten Liste von eingestuften potenziellen kausalen Genen und jedes aus der zweiten Liste von eingestuften potenziellen kausalen Genen durch Verwenden eines ungarischen Abgleichalgorithmus, um eine Mehrzahl von Eins-zu-Eins-Genotyp-Phänotyp-Übereinstimmungen zu erhalten, die jedem aus der Mehrzahl von Testpersonen entsprechen, basierend auf dem entsprechenden Ähnlichkeitsmaß, wobei der ungarische Abgleichalgorithmus die Genauigkeit des Abgleichs durch Berechnen einer Übereinstimmung durch Finden einer optimalen Zuordnung verbessert, die das Ähnlichkeitsmaß zwischen den Genotyp- und Phänotypfällen maximiert (312).

2. Verfahren nach Anspruch 1, wobei das Segmentieren der Mehrzahl von Phänotypen basierend auf der Mehrzahl von Metadaten Folgendes umfasst:
Segmentieren der Mehrzahl von Phänotypen, um einen Satz von männlichen Phänotypen und einen Satz von weiblichen Phänotypen basierend auf dem Geschlecht zu erhalten;
Segmentieren des Satzes von männlichen Phänotypen in eine Mehrzahl von männlichen ethnischen Phänotypen basierend auf der Ethnizität; und
gleichzeitiges Segmentieren des Satzes von weiblichen Phänotypen in eine Mehrzahl von weiblichen ethnischen Phänotypen basierend auf der Ethnizität.

3. Verfahren nach Anspruch 1, wobei das Segmentieren der Mehrzahl von Genotypsequenzen basierend auf der Mehrzahl von Metadaten Folgendes umfasst:
Segmentieren der Mehrzahl von Genotypsequenzen, um einen Satz von männlichen Genotypen und einen Satz von weiblichen Genotypen basierend auf dem Geschlecht zu erhalten;
Segmentieren des Satzes von männlichen Genotypen in eine Mehrzahl von männlichen ethnischen Genotypen basierend auf der Ethnizität; und
gleichzeitiges Segmentieren des Satzes von weiblichen Genotypen in eine Mehrzahl von weiblichen ethnischen Genotypen basierend auf der Ethnizität.

4. Verfahren nach Anspruch 1, wobei es wahrscheinlich ist, dass eine Variante des potenziellen Kausalgens einen Satz von Phänotypen verursacht, die einem Referenzsubjekt entsprechen.

5. System (100) zum Abgleichen klinischer Phänotypbeschreibungen und pathogener Varianten, das Folgendes umfasst:
mindestens einen Speicher (104), der programmierte Anweisungen speichert;
eine oder mehrere Eingabe/Ausgabe- (I/O-) Schnittstellen (112); und
einen oder mehrere Hardwareprozessoren (102), die betriebsfähig mit dem mindestens einen Speicher (104) gekoppelt sind, wobei der eine oder die mehreren Hardwareprozessoren (102) durch die programmierten Anweisungen konfiguriert sind zum:
Empfangen einer Mehrzahl von Phänotypen und einer Mehrzahl von Genotypsequenzen, die sich auf eine Mehrzahl von Testpersonen beziehen;
Segmentieren der Mehrzahl von Phänotypen und der Mehrzahl von Genotypsequenzen basierend auf einer Mehrzahl von Metadaten, wobei die Mehrzahl von Metadaten ein Geschlecht und eine Ethnizität umfasst, die mit der Testperson assoziiert sind;
Berechnen einer ersten Liste von eingestuften potenziellen kausalen Genen für die Mehrzahl von segmentierten Phänotypen basierend auf einer phänotypbasierten Genpriorisierung, wobei die erste Liste von eingestuften potenziellen kausalen Genen mit entsprechenden Phänotypen aus dem Satz von segmentierten Phänotypen assoziiert ist, wobei das Berechnen der ersten Liste von eingestuften potenziellen kausalen Genen basierend auf der phänotypbasierten Genpriorisierung die folgenden Schritte umfasst:
Bereitstellen der Mehrzahl von segmentierten Phänotypen als eine Eingabe in einen Phänotyp-Tagger, wobei jeder Phänotyp aus der Mehrzahl von Phänotypen mit einem anfänglichen menschlichen Phänotyp-Ontologie-, HPO-, Code assoziiert ist;
Bereitstellen zusätzlicher HPO-Codes als Ausgabe durch den Phänotyp-Tagger unter Verwendung eines Phänotyp-Wörterbuchs;
Erweitern der zusätzlichen HPO-Codes mit den anfänglichen HPO-Codes, um HPO-Codes zu erhalten; und
Abfragen eines heterogenen Assoziationsnetzwerks für seltene Krankheiten, HANRD, durch Verwenden der HPO-Codes, um die erste Liste von eingestuften potenziellen kausalen Genen zu erhalten, die mit den entsprechenden Phänotypen aus dem Satz von segmentierten Phänotypen assoziiert sind;
gleichzeitiges Berechnen einer zweiten Liste von eingestuften potenziellen kausalen Genen für die Mehrzahl von segmentierten Genotypen basierend auf einer Genomvariantenpriorisierung, wobei die Genomvariantenpriorisierung genomische Varianten analysiert, annotiert und priorisiert, wobei das Berechnen der zweiten Liste von eingestuften potenziellen kausalen Genen basierend auf der Genomvariantenpriorisierung die folgenden Schritte umfasst:
Bereitstellen einer Mehrzahl von Genotypvariantenaufrufformat-, VCF-, Dateien als eine Eingabe in eine Ensemble-Varianteneffekt-Priorisierungspipeline;
Ableiten einer Variantenbewertung durch Priorisieren von Varianten unter Verwendung eines internen Bewertungsschemas, das von 0 bis 1 reicht und gewichtete Kombinationen von Konservierungs- und Funktionsbewertungen darstellt, wobei die Konservierungsbewertungen verwendet werden, um eine Variante intern als eine von schädlich, möglicherweise schädlich oder toleriert zu klassifizieren, basierend auf Schwellenwerten, die von einer ClinVar-Datenbank abgeleitet werden; und
Durchführen der Variantenfilterung basierend auf einer Variantenbewertungsschwelle, wobei die Variantenfilterung Folgendes umfasst:
Auswählen einer Mehrzahl von hochwertigen Varianten durch Einbeziehen der Varianten mit PASS und der Variantenbewertungsschwelle;
Eliminieren der Varianten, die in polymorphen Genen aufgetreten sind;
Einstufen der Gene basierend auf den Varianten durch Kombinieren von Bewertungen der beiden besten Bewertungsvarianten in einem gleichen Gen für ein rezessives Vererbungsmodell und Auswählen des Gens mit einer besten Bewertungsvariante für das dominante Vererbungsmodell; und
Erzeugen der zweiten Liste von potenziellen kausalen Genen für die Mehrzahl von segmentierten Genotypen basierend auf der Einstufung;
Berechnen eines Ähnlichkeitsmaßes zwischen jedem aus der ersten Liste von eingestuften potenziellen kausalen Genen und jedem aus der zweiten Liste von eingestuften potenziellen kausalen Genen basierend auf einer ranggesteuerten Überlappung, wobei die ranggesteuerte Überlappung zwei eingestufte Listen unterschiedlicher Größe vergleicht; und
Abgleichen jedes aus der ersten Liste von eingestuften potenziellen kausalen Genen und jedes aus der zweiten Liste von eingestuften potenziellen kausalen Genen durch Verwenden eines ungarischen Abgleichalgorithmus, um eine Mehrzahl von Eins-zu-Eins-Genotyp-Phänotyp-Übereinstimmungen zu erhalten, die jedem aus der Mehrzahl von Testpersonen entsprechen, basierend auf dem entsprechenden Ähnlichkeitsmaß, wobei der ungarische Abgleichalgorithmus die Genauigkeit des Abgleichs durch Berechnen einer Übereinstimmung durch Finden einer optimalen Zuordnung verbessert, die das Ähnlichkeitsmaß zwischen den Genotyp- und Phänotypfällen maximiert.

6. System nach Anspruch 5, wobei das Segmentieren der Mehrzahl von Phänotypen basierend auf der Mehrzahl von Metadaten Folgendes umfasst:
Segmentieren der Mehrzahl von Phänotypen, um einen Satz von männlichen Phänotypen und einen Satz von weiblichen Phänotypen basierend auf dem Geschlecht zu erhalten;
Segmentieren des Satzes von männlichen Phänotypen in eine Mehrzahl von männlichen ethnischen Phänotypen basierend auf der Ethnizität; und
gleichzeitiges Segmentieren des Satzes von weiblichen Phänotypen in eine Mehrzahl von weiblichen ethnischen Phänotypen basierend auf der Ethnizität.

7. System nach Anspruch 5, wobei das Segmentieren der Mehrzahl von Genotypsequenzen basierend auf der Mehrzahl von Metadaten Folgendes umfasst:
Segmentieren der Mehrzahl von Genotypsequenzen, um einen Satz von männlichen Genotypen und einen Satz von weiblichen Genotypen basierend auf dem Geschlecht zu erhalten;
Segmentieren des Satzes von männlichen Genotypen in eine Mehrzahl von männlichen ethnischen Genotypen basierend auf der Ethnizität; und
gleichzeitiges Segmentieren des Satzes von weiblichen Genotypen in eine Mehrzahl von weiblichen ethnischen Genotypen basierend auf der Ethnizität.

8. System nach Anspruch 5, wobei es wahrscheinlich ist, dass eine Variante des potenziellen Kausalgens einen Satz von Phänotypen verursacht, die einem Referenzsubjekt entsprechen.

9. Ein oder mehrere nichtflüchtige maschinenlesbare Informationsspeichermedien, die eine oder mehrere Anweisungen umfassen, die bei Ausführung durch einen oder mehrere Hardwareprozessoren Folgendes bewirken:
Empfangen, durch einen oder mehrere Hardwareprozessoren, einer Mehrzahl von Phänotypen und einer Mehrzahl von Genotypsequenzen, die sich auf eine Mehrzahl von Testpersonen beziehen;
Segmentieren, durch den einen oder die mehreren Hardwareprozessoren, der Mehrzahl von Phänotypen und der Mehrzahl von Genotypsequenzen basierend auf einer Mehrzahl von Metadaten, wobei die Mehrzahl von Metadaten ein Geschlecht und eine Ethnizität umfasst, die mit der Testperson assoziiert sind;
Berechnen, durch den einen oder die mehreren Hardwareprozessoren, einer ersten Liste von eingestuften potenziellen kausalen Genen für die Mehrzahl von segmentierten Phänotypen basierend auf einer phänotypbasierten Genpriorisierung, wobei die erste Liste von eingestuften potenziellen kausalen Genen mit entsprechenden Phänotypen aus dem Satz von segmentierten Phänotypen assoziiert ist, wobei das Berechnen der ersten Liste von eingestuften potenziellen kausalen Genen basierend auf der phänotypbasierten Genpriorisierung die folgenden Schritte umfasst:
Bereitstellen der Mehrzahl von segmentierten Phänotypen als eine Eingabe in einen Phänotyp-Tagger, wobei jeder Phänotyp aus der Mehrzahl von Phänotypen mit einem anfänglichen menschlichen Phänotyp-Ontologie-, HPO-, Code assoziiert ist;
Bereitstellen zusätzlicher HPO-Codes als Ausgabe durch den Phänotyp-Tagger unter Verwendung eines Phänotyp-Wörterbuchs;
Erweitern der zusätzlichen HPO-Codes mit den anfänglichen HPO-Codes, um HPO-Codes zu erhalten; und
Abfragen eines heterogenen Assoziationsnetzwerks für seltene Krankheiten, HANRD, durch Verwenden der HPO-Codes, um die erste Liste von eingestuften potenziellen kausalen Genen zu erhalten, die mit den entsprechenden Phänotypen aus dem Satz von segmentierten Phänotypen assoziiert sind;
gleichzeitiges Berechnen, durch den einen oder die mehreren Hardwareprozessoren, einer zweiten Liste von eingestuften potenziellen kausalen Genen für die Mehrzahl von segmentierten Genotypen basierend auf einer Genomvariantenpriorisierung, wobei die Genomvariantenpriorisierung genomische Varianten analysiert, annotiert und priorisiert, wobei das Berechnen der zweiten Liste von eingestuften potenziellen kausalen Genen basierend auf der Genomvariantenpriorisierung die folgenden Schritte umfasst:
Bereitstellen einer Mehrzahl von Genotypvariantenaufrufformat-, VCF-, Dateien als eine Eingabe in eine Ensemble-Varianteneffekt-Priorisierungspipeline;
Ableiten einer Variantenbewertung durch Priorisieren von Varianten unter Verwendung eines internen Bewertungsschemas, das von 0 bis 1 reicht und gewichtete Kombinationen von Konservierungs- und Funktionsbewertungen darstellt, wobei die Konservierungsbewertungen verwendet werden, um eine Variante intern als eine von schädlich, möglicherweise schädlich oder toleriert zu klassifizieren, basierend auf Schwellenwerten, die von einer ClinVar-Datenbank abgeleitet werden; und
Durchführen der Variantenfilterung basierend auf einer Variantenbewertungsschwelle, wobei die Variantenfilterung Folgendes umfasst:
Auswählen einer Mehrzahl von hochwertigen Varianten durch Einbeziehen der Varianten mit PASS und der Variantenbewertungsschwelle;
Eliminieren der Varianten, die in polymorphen Genen aufgetreten sind;
Einstufen der Gene basierend auf den Varianten durch Kombinieren von Bewertungen der beiden besten Bewertungsvarianten in einem gleichen Gen für ein rezessives Vererbungsmodell und Auswählen des Gens mit einer besten Bewertungsvariante für das dominante Vererbungsmodell; und
Erzeugen der zweiten Liste von potenziellen kausalen Genen für die Mehrzahl von segmentierten Genotypen basierend auf der Einstufung;
Berechnen, durch den einen oder die mehreren Hardwareprozessoren, eines Ähnlichkeitsmaßes zwischen jedem aus der ersten Liste von eingestuften potenziellen kausalen Genen und jedem aus der zweiten Liste von eingestuften potenziellen kausalen Genen basierend auf einer ranggesteuerten Überlappung, wobei die ranggesteuerte Überlappung zwei eingestufte Listen unterschiedlicher Größe vergleicht; und
Abgleichen, durch den einen oder die mehreren Hardwareprozessoren, jedes aus der ersten Liste von eingestuften potenziellen kausalen Genen und jedes aus der zweiten Liste von eingestuften potenziellen kausalen Genen durch Verwenden eines ungarischen Abgleichalgorithmus, um eine Mehrzahl von Eins-zu-Eins-Genotyp-Phänotyp-Übereinstimmungen zu erhalten, die jedem aus der Mehrzahl von Testpersonen entsprechen, basierend auf dem entsprechenden Ähnlichkeitsmaß, wobei der ungarische Abgleichalgorithmus die Genauigkeit des Abgleichs durch Berechnen einer Übereinstimmung durch Finden einer optimalen Zuordnung verbessert, die das Ähnlichkeitsmaß zwischen den Genotyp- und Phänotypfällen maximiert.

## Revendications

1. Procédé mis en œuvre par processeur pour mettre en correspondance des descriptions de phénotypes cliniques et des variants pathogènes, le procédé comprenant :
la réception, par un ou plusieurs processeurs matériels, d'une pluralité de phénotypes et d'une pluralité de séquences génotypiques se rapportant à une pluralité de sujets à tester (302) ;
la segmentation, par les un ou plusieurs processeurs matériels, de la pluralité de phénotypes et de la pluralité de séquences génotypiques sur la base d'une pluralité de métadonnées, dans lequel la pluralité de métadonnées comprend un genre et une ethnicité associés au sujet à tester (304) ;
le calcul, par les un ou plusieurs processeurs matériels, d'une première liste de gènes causaux potentiels classés pour la pluralité de phénotypes segmentés sur la base d'une priorisation génique basée sur un phénotype, dans lequel la première liste de gènes causaux potentiels classés est associée à des phénotypes correspondants de l'ensemble de phénotypes segmentés (306), dans lequel le calcul de la première liste de gènes causaux potentiels classés sur la base de la priorisation génique basée sur un phénotype comprend les étapes de :
fourniture de la pluralité de phénotypes segmentés en tant qu'entrée à un marqueur de phénotype, dans lequel chaque phénotype de la pluralité de phénotypes est associé à un code d'ontologie de phénotype humain, HPO, initial ;
fourniture de codes HPO supplémentaires en tant que sortie par le marqueur de phénotype en utilisant un dictionnaire de phénotypes ;
augmentation des codes HPO supplémentaires avec les codes HPO initiaux pour obtenir des codes HPO ; et
interrogation d'un réseau d'association hétérogène pour maladies rares, HANRD, en utilisant les codes HPO pour obtenir la première liste de gènes causaux potentiels classés associés aux phénotypes correspondants de l'ensemble de phénotypes segmentés ;
calcul simultané, par les un ou plusieurs processeurs matériels, d'une seconde liste de gènes causaux potentiels classés pour la pluralité de génotypes segmentés sur la base d'une priorisation de variant génomique, dans lequel la priorisation de variant génomique analyse, annote et priorise des variants génomiques (308), dans lequel le calcul de la seconde liste de gènes causaux potentiels classés sur la base de la priorisation de variant génomique comprend les étapes de :
fourniture d'une pluralité de fichiers de format d'appel de variant de génotype, VCF, en tant qu'entrée à un pipeline de priorisation d'effet de variant d'ensemble ;
dérivation d'un score de variant en priorisant des variants en utilisant un schéma de notation interne, allant de 0 à 1 et représentant une combinaison pondérée de scores de conservation et fonctionnels, dans lequel les scores de conservation sont utilisés pour classer en interne un variant comme étant l'un parmi endommageant, éventuellement endommageant ou toléré, sur la base de seuils dérivés d'une base de données ClinVar ; et
réalisation du filtrage de variant sur la base d'un seuil de score de variant, dans lequel le filtrage de variant comprend :
sélection d'une pluralité de variants de haute qualité en incluant les variants ayant un PASS et le seuil de score de variant ;
élimination des variants survenus dans des gènes polymorphes ;
classement des gènes sur la base des variants en combinant des scores des deux variants de notation supérieurs dans un même gène pour un modèle d'héritage récessif et sélection du gène avec un variant de notation supérieur pour le modèle d'héritage dominant ; et
génération de la seconde liste de gènes causaux potentiels pour la pluralité de génotypes segmentés sur la base du classement ;
calcul, par les un ou plusieurs processeurs matériels, d'une mesure de similarité entre chacun de la première liste de gènes causaux potentiels classés et chacun de la seconde liste de gènes causaux potentiels classés sur la base d'un chevauchement biaisé par classement, dans lequel le chevauchement biaisé par classement compare deux listes classées de taille différente (310) ; et
mise en correspondance, par les un ou plusieurs processeurs matériels, de chacun de la première liste de gènes causaux potentiels classés et de chacun de la seconde liste de gènes causaux potentiels classés en utilisant un algorithme de mise en correspondance hongrois pour obtenir une pluralité de correspondance génotypique-phénotype un à un correspondant à chacun de la pluralité de sujets à tester la base de la mesure de similarité correspondante, dans lequel l'algorithme de mise en correspondance hongrois améliore la précision de la mise en correspondance en calculant une correspondance en trouvant une attribution optimale qui maximise la mesure de similarité entre les cas de génotype et de phénotype (312).

2. Procédé selon la revendication 1, dans lequel la segmentation de la pluralité de phénotypes sur la base de la pluralité de métadonnées comprend :
la segmentation de la pluralité de phénotypes pour obtenir un ensemble de phénotypes mâles et un ensemble de phénotypes femelles sur la base du genre ;
la segmentation de l'ensemble de phénotypes mâles en une pluralité de phénotypes ethniques mâles sur la base de l'ethnicité ; et
la segmentation simultanée de l'ensemble de phénotypes femelles en une pluralité de phénotypes ethniques femelles sur la base de l'ethnicité.

3. Procédé selon la revendication 1, dans lequel la segmentation de la pluralité de séquences génotypiques sur la base de la pluralité de métadonnées comprend :
la segmentation de la pluralité de séquences génotypiques pour obtenir un ensemble de génotypes mâles et un ensemble de génotypes femelles sur la base du genre ;
la segmentation de l'ensemble de génotypes mâles en une pluralité de génotypes ethniques mâles sur la base de l'ethnicité ; et
la segmentation simultanée de l'ensemble de génotypes femelles en une pluralité de génotypes ethniques femelles sur la base de l'ethnicité.

4. Procédé selon la revendication 1, dans lequel un variant du gène causal potentiel est susceptible de provoquer un ensemble de phénotypes correspondant à un sujet de référence.

5. Système (100) pour mettre en correspondance des descriptions de phénotypes cliniques et des variants pathogènes, comprenant :
au moins une mémoire (104) stockant des instructions programmées ;
une ou plusieurs interfaces d'entrée/sortie (E/S) (112) ; et
un ou plusieurs processeurs matériels (102) couplés de manière opérationnelle à l'au moins une mémoire (104), dans lequel les un ou plusieurs processeurs matériels (102) sont configurés par les instructions programmées pour :
recevoir une pluralité de phénotypes et une pluralité de séquences génotypiques se rapportant à une pluralité de sujets à tester ;
segmenter la pluralité de phénotypes et la pluralité de séquences génotypiques sur la base d'une pluralité de métadonnées, dans lequel la pluralité de métadonnées comprend un genre et une ethnicité associés au sujet à tester ;
calculer une première liste de gènes causaux potentiels classés pour la pluralité de phénotypes segmentés sur la base d'une priorisation génique basée sur un phénotype, dans lequel la première liste de gènes causaux potentiels classés est associée à des phénotypes correspondants de l'ensemble de phénotypes segmentés, dans lequel le calcul de la première liste de gènes causaux potentiels classés sur la base de la priorisation génique basée sur un phénotype comprend les étapes de :
fourniture de la pluralité de phénotypes segmentés en tant qu'entrée à un marqueur de phénotype, dans lequel chaque phénotype de la pluralité de phénotypes est associé à un code d'ontologie de phénotype humain, HPO, initial ;
fourniture de codes HPO supplémentaires en tant que sortie par le marqueur de phénotype en utilisant un dictionnaire de phénotypes ;
augmentation des codes HPO supplémentaires avec les codes HPO initiaux pour obtenir des codes HPO ; et
interrogation d'un réseau d'association hétérogène pour maladies rares, HANRD, en utilisant les codes HPO pour obtenir la première liste de gènes causaux potentiels classés associés aux phénotypes correspondants de l'ensemble de phénotypes segmentés ;
calcul simultané d'une seconde liste de gènes causaux potentiels classés pour la pluralité de génotypes segmentés sur la base d'une priorisation de variant génomique, dans lequel la priorisation de variant génomique analyse, annote et priorise des variants génomiques, dans lequel le calcul de la seconde liste de gènes causaux potentiels classés sur la base de la priorisation de variant génomique comprend les étapes de :
fourniture d'une pluralité de fichiers de format d'appel de variant de génotype, VCF, en tant qu'entrée à un pipeline de priorisation d'effet de variant d'ensemble ;
dérivation d'un score de variant en priorisant des variants en utilisant un schéma de notation interne, allant de 0 à 1 et représentant une combinaison pondérée de scores de conservation et fonctionnels, dans lequel les scores de conservation sont utilisés pour classer en interne un variant comme étant l'un parmi endommageant, éventuellement endommageant ou toléré, sur la base de seuils dérivés d'une base de données ClinVar ; et
réalisation du filtrage de variant sur la base d'un seuil de score de variant, dans lequel le filtrage de variant comprend :
sélection d'une pluralité de variants de haute qualité en incluant les variants ayant un PASS et le seuil de score de variant ;
élimination des variants survenus dans des gènes polymorphes ;
classement des gènes sur la base des variants en combinant des scores des deux variants de notation supérieurs dans un même gène pour un modèle d'héritage récessif et sélection du gène avec un variant de notation supérieur pour le modèle d'héritage dominant ; et
génération de la seconde liste de gènes causaux potentiels pour la pluralité de génotypes segmentés sur la base du classement ;
calcul d'une mesure de similarité entre chacun de la première liste de gènes causaux potentiels classés et chacun de la seconde liste de gènes causaux potentiels classés sur la base d'un chevauchement biaisé par classement, dans lequel le chevauchement biaisé par classement compare deux listes classées de taille différente ; et
mise en correspondance de chacun de la première liste de gènes causaux potentiels classés et de chacun de la seconde liste de gènes causaux potentiels classés en utilisant un algorithme de mise en correspondance hongrois pour obtenir une pluralité de correspondances génotype-phénotype un à un correspondant à chacun de la pluralité de sujets à tester sur la base de la mesure de similarité correspondante, dans lequel l'algorithme de mise en correspondance hongrois améliore la précision de la mise en correspondance en calculant une correspondance en trouvant une attribution optimale qui maximise la mesure de similarité entre les cas de génotype et de phénotype.

6. Système selon la revendication 5, dans lequel la segmentation de la pluralité de phénotypes sur la base de la pluralité de métadonnées comprend :
la segmentation de la pluralité de phénotypes pour obtenir un ensemble de phénotypes mâles et un ensemble de phénotypes femelles sur la base du genre ;
la segmentation de l'ensemble de phénotypes mâles en une pluralité de phénotypes ethniques mâles sur la base de l'ethnicité ; et
la segmentation simultanée de l'ensemble de phénotypes femelles en une pluralité de phénotypes ethniques femelles sur la base de l'ethnicité.

7. Système selon la revendication 5, dans lequel la segmentation de la pluralité de séquences génotypiques sur la base de la pluralité de métadonnées comprend :
la segmentation de la pluralité de séquences génotypiques pour obtenir un ensemble de génotypes mâles et un ensemble de génotypes femelles sur la base du genre ;
la segmentation de l'ensemble de génotypes mâles en une pluralité de génotypes ethniques mâles sur la base de l'ethnicité ; et
la segmentation simultanée de l'ensemble de génotypes femelles en une pluralité de génotypes ethniques femelles sur la base de l'ethnicité.

8. Système selon la revendication 5, dans lequel un variant du gène causal potentiel est susceptible de provoquer un ensemble de phénotypes correspondant à un sujet de référence.

9. Un ou plusieurs supports de stockage d'informations lisibles par machine non transitoires comprenant une ou plusieurs instructions qui, lorsqu'elles sont exécutées par un ou plusieurs processeurs matériels, provoquent :
la réception, par un ou plusieurs processeurs matériels, d'une pluralité de phénotypes et d'une pluralité de séquences génotypiques se rapportant à une pluralité de sujets à tester ;
la segmentation, par les un ou plusieurs processeurs matériels, de la pluralité de phénotypes et de la pluralité de séquences génotypiques sur la base d'une pluralité de métadonnées, dans lequel la pluralité de métadonnées comprend un genre et une ethnicité associés au sujet à tester ;
le calcul, par les un ou plusieurs processeurs matériels, d'une première liste de gènes causaux potentiels classés pour la pluralité de phénotypes segmentés sur la base d'une priorisation génique basée sur un phénotype, dans lequel la première liste de gènes causaux potentiels classés est associée à des phénotypes correspondants de l'ensemble de phénotypes segmentés, dans lequel le calcul de la première liste de gènes causaux potentiels classés sur la base de la priorisation génique basée sur un phénotype comprend les étapes de :
fourniture de la pluralité de phénotypes segmentés en tant qu'entrée à un marqueur de phénotype, dans lequel chaque phénotype de la pluralité de phénotypes est associé à un code d'ontologie de phénotype humain, HPO, initial ;
fourniture de codes HPO supplémentaires en tant que sortie par le marqueur de phénotype en utilisant un dictionnaire de phénotypes ;
augmentation des codes HPO supplémentaires avec les codes HPO initiaux pour obtenir des codes HPO ; et
interrogation d'un réseau d'association hétérogène pour maladies rares, HANRD, en utilisant les codes HPO pour obtenir la première liste de gènes causaux potentiels classés associés aux phénotypes correspondants de l'ensemble de phénotypes segmentés ;
calcul simultané, par les un ou plusieurs processeurs matériels, d'une seconde liste de gènes causaux potentiels classés pour la pluralité de génotypes segmentés sur la base d'une priorisation de variant génomique, dans lequel la priorisation de variant génomique analyse, annote et priorise des variants génomiques, dans lequel le calcul de la seconde liste de gènes causaux potentiels classés sur la base de la priorisation de variant génomique comprend les étapes de :
fourniture d'une pluralité de fichiers de format d'appel de variant de génotype, VCF, en tant qu'entrée à un pipeline de priorisation d'effet de variant d'ensemble ;
dérivation d'un score de variant en priorisant des variants en utilisant un schéma de notation interne, allant de 0 à 1 et représentant une combinaison pondérée de scores de conservation et fonctionnels, dans lequel les scores de conservation sont utilisés pour classer en interne un variant comme étant l'un parmi endommageant, éventuellement endommageant ou toléré, sur la base de seuils dérivés d'une base de données ClinVar ; et
réalisation du filtrage de variant sur la base d'un seuil de score de variant, dans lequel le filtrage de variant comprend :
sélection d'une pluralité de variants de haute qualité en incluant les variants ayant un PASS et le seuil de score de variant ;
élimination des variants survenus dans des gènes polymorphes ;
classement des gènes sur la base des variants en combinant des scores des deux variants de notation supérieurs dans un même gène pour un modèle d'héritage récessif et sélection du gène avec un variant de notation supérieur pour le modèle d'héritage dominant ; et
génération de la seconde liste de gènes causaux potentiels pour la pluralité de génotypes segmentés sur la base du classement ;
calcul, par les un ou plusieurs processeurs matériels, d'une mesure de similarité entre chacun de la première liste de gènes causaux potentiels classés et chacun de la seconde liste de gènes causaux potentiels classés sur la base d'un chevauchement biaisé par classement, dans lequel le chevauchement biaisé par classement compare deux listes classées de taille différente ; et
mise en correspondance, par les un ou plusieurs processeurs matériels, de chacun de la première liste de gènes causaux potentiels classés et de chacun de la seconde liste de gènes causaux potentiels classés en utilisant un algorithme de mise en correspondance hongrois pour obtenir une pluralité de correspondances génotype-phénotype un à un correspondant à chacun de la pluralité de sujets à tester sur la base de la mesure de similarité correspondante, dans lequel l'algorithme de mise en correspondance hongrois améliore la précision de la mise en correspondance en calculant une correspondance en trouvant une attribution optimale qui maximise la mesure de similarité entre les cas de génotype et de phénotype.
